# FASCICULE DE BREVET EUROPEEN

(11) **EP 1 899 048 B1**
(45) Date de publication et mention de la délivrance du brevet: **17.12.2008**
(21) Numéro de dépôt: 05786105.6
(22) Date de dépôt: 01.07.2005
(51) Int. Cl.: B01J 19/08, B01L 3/00, C07F 7/18, C23C 16/513, C12Q 1/02, C07B 47/00, G01N 33/53

(54) **REVETEMENT DE SURFACE HYDROPHOBE ET A FAIBLE HYSTERESIS DE MOUILLAGE, PROCEDE DE DEPOT D'UN TEL REVETEMENT, MICRO-COMPOSANT ET UTILISATION**
HYDROPHOBE OBERFLÄCHENBESCHICHTUNG MIT GERINGER BENETZUNGSHYSTERESE, AUFBRINGUNGSVERFAHREN DAFÜR, MIKROKOMPONENTE UND VERWENDUNG
LOW WETTING HYSTERESIS HYDROPHOBIC SURFACE COATING, METHOD FOR DEPOSITING SAME, MICROCOMPONENT AND USE

(43) Date de publication de la demande: 19.03.2008
(73) Titulaire: COMMISSARIAT A L'ENERGIE ATOMIQUE, 75015 Paris (FR)
(72) Inventeur: PLISSONNIER, Marc, F-38320 Eybens (FR); GAILLARD, Frédéric, F-38500 Voiron (FR); FOUILLET, Yves, F-38340 Voreppe (FR)
(74) Mandataire: Jouvray, Marie-Andrée
(86) Numéro de dépôt international: PCT/FR2005/001694
(87) Numéro de publication internationale: WO 2007/003720

(56) Documents cités:
- EP-A- 0 289 402
- EP-A- 1 011 298
- WO-A-97/12966
- US-A- 4 243 692
- US-A- 5 718 967
- BENITEZ F ET AL: "Improvement of hardness in plasma polymerized hexamethyldisiloxane coatings by silica-like surface modification" THIN SOLID FILMS, ELSEVIER-SEQUOIA S.A. LAUSANNE, CH, vol. 377-378, 1 décembre 2000 (2000-12-01), pages 109-114, XP004226677 ISSN: 0040-6090
- HEGEMANN D ET AL: "Improving the adhesion of siloxane-based plasma coatings on polymers with defined wetting properties" CAPLUS, 23 avril 2003 (2003-04-23), XP002326575

## Description

### Domaine technique de l'invention

L'invention concerne un revêtement de surface hydrophobe.

L'invention concerne également un procédé de dépôt d'un tel revêtement de surface, un micro-composant comportant au moins un substrat muni d'un réseau d'électrodes et dont une face est destinée à recevoir une goutte de liquide et l'utilisation d'un tel micro-composant.

### État de la technique

Dans de nombreux domaines mettant en jeu des solutions aqueuses à analyser, tels que le domaine des analyses chimiques, biologiques, biochimiques et environnementales, le domaine médical ou le contrôle qualité, le développement des micro-technologies a permis de réaliser des micro-composants formant des laboratoires sur puces ou "lab-on-chip". De tels laboratoires sur puces permettent de miniaturiser les volumes de liquide à analyser, tout en augmentant la vitesse et la sensibilité des mesures. Ces micro-composants comportent généralement, des canaux ou des cavités qui peuvent générer des interactions entre les solutés et les parois des canaux ou des cavités ainsi que des phénomènes d'absorption d'entités réactives limitant les rendements de réaction ou formant des sources de contamination.

Il a, alors, été proposé de réaliser des micro-composants s'affranchissant des canaux ou des parois, en permettant à chaque goutte de liquide, disposée sur une surface libre d'un micro-composant, de former un micro-réacteur indépendant et de se déplacer librement d'un point à un autre de la surface libre. Un des modes de déplacement et/ou de manipulation des gouttes repose, par exemple, sur le principe d'électromouillage sur diélectrique, plus connu sous le nom anglo-saxon "Electrowetting-on-dielectric" (EWOD).

Le principe de l'électromouillage sur diélectrique consiste à déposer une goutte sur un substrat comportant un premier réseau d'électrodes et recouvert d'un revêtement isolant et hydrophobe. Un second réseau d'électrodes est disposé en regard du premier réseau, au-dessus de la goutte de manière à appliquer localement une tension entre deux électrodes des premier et second réseaux. De plus, la surface de la zone du revêtement où est appliquée la tension forme une capacité, elle se charge et attire la goutte en créant une force provoquant le mouvement ou l'étalement de la goutte. Il est, alors possible, de proche en proche, de déplacer des liquides et de les mélanger. Le principe de l'électromouillage est également utilisé dans des micro-composants comportant des canaux ou des cavités, pour faciliter, par exemple, le déplacement du liquide à l'intérieur du canal.

Cependant, le principe d'électromouillage nécessite que la surface libre sur laquelle est disposée la goutte soit très hydrophobe. L'hydrophobicité d'une surface est définie par l'angle de contact entre la surface et le ménisque d'une goutte d'eau disposée sur ladite surface. Plus la différence entre l'angle de contact à l'état initial, c'est-à-dire avant l'application de la force de déplacement ou d'étalement, et celui obtenu lorsque la force est appliquée, est grande, plus le déplacement est important. De plus, plus l'angle de contact, à l'état initial, est élevé, plus la force nécessaire au déplacement ou à l'étalement de la goutte est faible. Ainsi, pour obtenir un déplacement significatif, il est, généralement nécessaire d'obtenir un angle de contact supérieur ou égal à 100°.

Le déplacement, la manipulation ou la déformation d'une goutte doit également être sensiblement réversible, c'est-à-dire que, lorsque l'on cesse d'appliquer la force provoquant le mouvement ou la déformation de la goutte, le système composé de la surface hydrophobe et de la goutte disposée sur ladite surface doit être dans un état le plus proche possible de l'état initial. Cette réversibilité dépend essentiellement d'un phénomène appelé hystérésis de mouillage, dépendant lui-même de la densité, de l'uniformité d'épaisseur, de la rugosité et l'homogénéité chimique de la surface. L'hystérésis de mouillage d'une surface détermine, en effet, l'état du système, après l'application de la force d'étalement ou de déplacement, ce qui permet de déterminer si un deuxième étalement ou déplacement peut être réalisé. Cependant, les paramètres d'hydrophobicité et d'hystérésis de mouillage étant liés, plus une surface est hydrophobe, moins l'hystérésis de mouillage a un effet important sur le déplacement de la goutte.

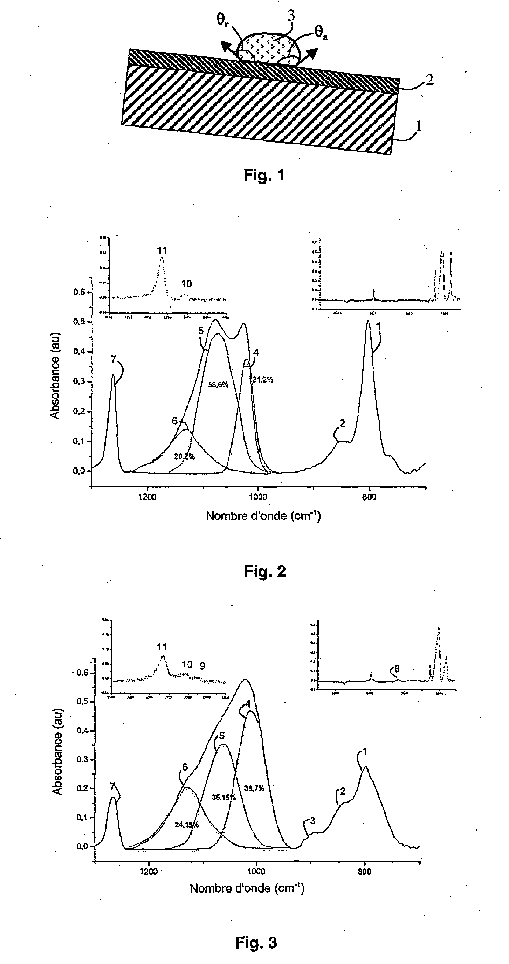

L'hystérésis de mouillage d'une surface ou hystérésis de mouillage-démouillage correspond, en effet, à un refus de mouiller une surface sèche, lorsque la goutte glisse sur ladite surface, ce phénomène se traduisant alors par une augmentation de l'angle de contact du côté où la goutte avance, également appelé angle d'avancée θₐ. De même, une surface préalablement mouillée tend à retenir la goutte, ce qui génère un angle de contact plus faible du côté où la goutte recule, également appelé angle de recul θᵣ. A titre d'illustration, les angles d'avancée θₐ et de recul θᵣ sont représentés à la figure 1 où un substrat 1 incliné comporte un revêtement de surface 2 hydrophobe sur lequel est disposé une goutte 3. Ainsi, on détermine l'hystérésis de mouillage du revêtement de surface 2 en mesurant l'écart entre l'angle maximum d'avancée θ_{a max} et l'angle minimum de recul θ_{r min}. Plus cet écart est important, plus l'hystérésis de mouillage du revêtement de surface est importante et plus la goutte d'eau se déplace difficilement. Généralement, dans le domaine de l'électromouillage sur diélectrique, il est souhaitable d'obtenir un revêtement de surface hydrophobe, ayant une hystérésis de mouillage inférieure ou égale à 15°.

Les revêtements hydrophobes utilisés pour former la surface libre des micro-composants adaptés à l'électromouillage sur diélectrique sont, généralement, composés de polymère fluoré liquide tel que le Téflon® commercialisé par la société DuPont. Or, bien que le Téflon® soit très hydrophobe, avec un angle de contact égal à 110°, sa valeur de constante diélectrique relative est de l'ordre de 2 et il présente un coefficient de claquage bas. Pour remédier à un coefficient de claquage bas, le revêtement de surface en Téflon® est généralement épais et la tension nécessaire pour déplacer une goutte est élevée. Par ailleurs, une couche mince diélectrique est disposée entre le revêtement en Téflon® et la surface du micro-composant, pour compenser la faible constante diélectrique du Téflon®. Enfin, le Téflon® présente l'inconvénient d'être généralement déposé à la tournette. Or, cette technique n'est applicable qu'aux surfaces parfaitement planes, ce qui limite l'utilisation d'un revêtement en Téflon® dans le domaine de l'électromouillage sur diélectrique.

Il a été proposé de remplacer le Téflon® par du Parylène car celui-ci peut être déposé par évaporation sous vide. Cette technique permet, en effet, d'obtenir un revêtement ayant une épaisseur relativement uniforme, quelle que soit la géométrie de la surface sur laquelle il est appliqué. De plus, le Parylène possède une constante diélectrique satisfaisante et une bonne résistance au claquage. Par contre, ce composé n'est pas suffisamment hydrophobe, l'angle de contact étant égal à 90°, et il comporte une trop grande hystérésis de mouillage pour permettre son utilisation dans un dispositif utilisant le phénomène d'électromouillage sur diélectrique.

### Objet de l'invention

L'invention a pour but d'obtenir un revêtement de surface hydrophobe remédiant aux inconvénients ci-dessus et ayant, plus particulièrement, une hystérésis de mouillage relativement faible, de préférence inférieure à 15° tout en conservant une bonne hydrophobicité et une constante diélectrique relative élevée.

Selon l'invention, ce but est atteint par le fait que le revêtement de surface hydrophobe comporte au moins une couche mince supérieure constituée par un composé choisi parmi SiCₓO_{y}:H avec x compris entre 1,4 et 2 et y compris entre 0,8 et 1,4 et SiC_{x'}N_{y'}:H avec x' compris entre 1,2 et 1,4 et y' compris entre 0,6 et 0,8, de manière à obtenir une surface libre à faible hystérésis de mouillage.

Selon un développement de l'invention, la couche mince supérieure a une épaisseur inférieure ou égale à 1 micromètre.

Selon un mode de réalisation préférentiel, le revêtement de surface comporte une couche mince inférieure diélectrique.

Selon une autre caractéristique de l'invention, la couche mince inférieure a une constante diélectrique relative supérieure ou égale à 2.

L'invention a également pour but un procédé de dépôt d'un tel revêtement de surface, sur une face d'un micro-composant, facile à mettre en oeuvre et permettant d'obtenir un revêtement de surface ayant une épaisseur constante, quelle que soit la géométrie du micro-composant.

Selon l'invention, ce but est atteint par le fait que le procédé de dépôt consiste au moins à réaliser un dépôt chimique en phase vapeur sur la face dudit micro-composant, pour former une couche mince supérieure constituée par un composé choisi parmi SiCₓO_{y}:H avec x compris entre 1,4 et 2 et y compris entre 0,8 et 1,4 et SiC_{x'}N_{y'}:H avec x' compris entre 1,2 et 1,4 et y' compris entre 0,6 et 0,8, le dépôt chimique en phase vapeur étant réalisé au moyen d'un précurseur choisi parmi les organo-silanes.

Selon un développement de l'invention, le dépôt chimique en phase vapeur est un dépôt chimique en phase vapeur assisté par plasma et, de préférence, réalisé en mode pulsé.

L'invention a également pour but un micro-composant comportant au moins un substrat muni d'un réseau d'électrodes et dont une face est destinée à recevoir au moins une goutte de liquide capable de se déplacer et/ou de se déformer facilement et rapidement sur la face du micro-composant.

Selon l'invention, ce but est atteint par le fait que le micro-composant comporte un revêtement de surface selon l'invention et disposé sur la face du micro-composant de manière à ce que la surface libre du revêtement reçoive la goutte.

Un tel micro-composant peut alors être utilisé en appliquant successivement une tension aux différentes électrodes afin de déplacer la goutte disposée sur la surface libre du revêtement.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
La figure 1 représente, en coupe, un substrat incliné comportant un revêtement de surface sur lequel est disposée une goutte.
Les figures 2 et 3 représentent les spectres infrarouges de deux composés SiC_{1,6}O_{1,2}:H, respectivement en conformation A et B.
Les figures 4 et 5 représentent graphiquement les hystérésis de mouillage de deux revêtements de surface composés de Téflon®, respectivement déposés à la tournette et par PECVD.
Les figures 6 et 7 représentent graphiquement les hystérésis de mouillage de deux revêtements de surface composés de SiC_{1,6}O_{1,2}:H, respectivement en conformation A et B et déposés par PECVD.
Les figures 8 et 9 représentent respectivement, en coupe et schématiquement, des premier et second revêtements de surface hydrophobes selon l'invention, disposés sur un micro-composant.
Les figures 10 à 17 représentent les différentes étapes de fabrication d'un micro-composant selon l'invention.

### Description de modes particuliers de réalisation

Selon l'invention, un revêtement de surface hydrophobe comporte au moins une couche supérieure constituée par un composé choisi parmi SiCₓO_{y}:H et SiC_{x'}N_{y'}:H, avec x compris entre 1,4 et 2 et y compris entre 0,8 et 1,4 et avec x' compris entre 1,2 et 1,4 et y' compris entre 0,6 et 0,8. De préférence, la couche mince supérieure a une épaisseur inférieure ou égale à 1 micromètre.

Par SiCₓO_{y}:H, on entend un composé choisi parmi les oxydes de silicium amorphes hydrogénés dopés par du carbone, la proportion atomique de carbone par rapport à la proportion atomique en oxygène correspondant au rapport x/y.

Par SiC_{x'}N_{y'}:H, on entend un composé choisi parmi les nitrures de silicium amorphes hydrogénés dopés par du carbone, la proportion atomique de carbone par rapport à la proportion atomique en azote correspondant au rapport x'/y'.

Un tel choix de composés, aptes à former la couche supérieure du revêtement de surface hydrophobe, est déterminé par la nature des liaisons chimiques présentes dans les composés. En effet, SICₓO_{y}:H et SiC_{x'}N_{y'}:H comportent chacun, des liaisons chimiques de type Si-CH₃, c'est-à-dire les liaisons chimiques présentes entre un atome de silicium et un groupement -CH₃. Or, les liaisons de type Si-CH₃ rendent la surface libre de la couche supérieure, particulièrement hydrophobe et lui confère une faible hystérésis de mouillage. Par ailleurs, l'atome d'oxygène ou l'atome d'azote, présent dans le composé, apporte à la couche mince une constante diélectrique relative élevée, de préférence supérieure ou égale à 2 et une bonne tenue au claquage. De plus, les composés choisis parmi SiCₓO_{y}:H et SiC_{x'}N_{y'}:H sont biocompatibles, les couches minces constituées par de tels composés étant alors adaptées aux micro-composants utilisés dans le domaine médical ou biologique.

Dans un mode particulier de réalisation, le revêtement de surface est déposé sur la face d'un micro-composant, en formant au moins la couche mince supérieure, sur la face du micro-composant. La formation de la couche mince supérieure est réalisée par dépôt chimique en phase vapeur, au moyen d'un précurseur choisi parmi les organo-silanes. Le dépôt chimique en phase vapeur est, de préférence, un dépôt chimique en phase vapeur assisté par plasma, également appelé PECVD, et plus particulièrement en mode pulsé. Le dépôt PECVD en mode pulsé permet, en effet, de modifier la rugosité d'une surface et donc de diminuer, de manière significative, l'hystérésis de mouillage de la surface. Pour une couche mince supérieure en SiCₓO_{y}:H, le précurseur organo-silane est, par exemple, l'octaméthylcyclotétrasiloxane (OMCTS) et il est mélangé avec de l'hélium. Pour une couche mince supérieure en SiC_{x'}N_{y'}:H, le précurseur organo-silane est, par exemple, le tétraméthylsilane (TMS), mélangé avec de l'azote.

Ainsi, à titre d'exemple, la couche mince supérieure est déposée sur la face d'un micro-composant disposé dans un dispositif de dépôt sous vide. Le dispositif de dépôt sous vide est, par exemple, une enceinte de dépôt chimique en phase vapeur assisté par un plasma excité par une décharge capacitive à 13,56 MHz. Un générateur basse fréquence ou radiofréquence permet alors de réaliser une décharge plasma au voisinage du micro-composant et le précurseur, mélangé avec de l'hélium ou de l'azote, est injecté dans l'enceinte, à pression réduite.

La réalisation d'un tel revêtement de surface permet, notamment, d'utiliser des technologies compatibles avec les techniques de la microélectronique, de réaliser des dépôts de revêtement sur de très grandes surfaces et de diminuer les étapes de fabrication d'un micro-composant adapté à l'électromouillage sur diélectrique. Par ailleurs, le dépôt chimique en phase vapeur permet de garantir une structure chimique particulière au composé de la couche mince supérieure, en formant notamment des liaisons chimiques de type Si-CH₃. De plus, le choix des composés formant la couche mince supérieure permet d'utiliser un procédé de dépôt, facile à mettre en oeuvre et permettant de réaliser un revêtement de surface ayant une épaisseur constante quelle que soit la géométrie du micro-composant, contrairement à d'autres composés utilisés pour le déplacement de liquide tel que le Téflon®. Ceci est particulièrement adapté aux micro-composants comportant une cavité ou un canal d'une profondeur de plusieurs centaines de micromètres d'épaisseur.

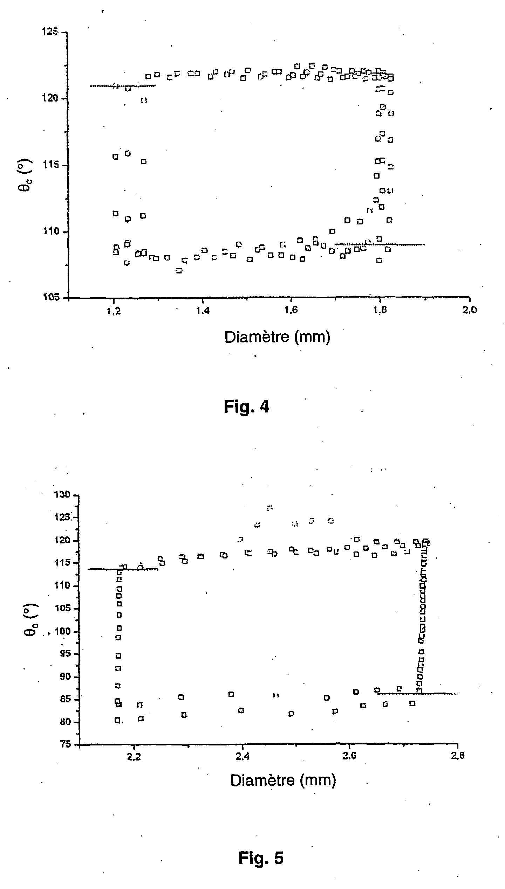

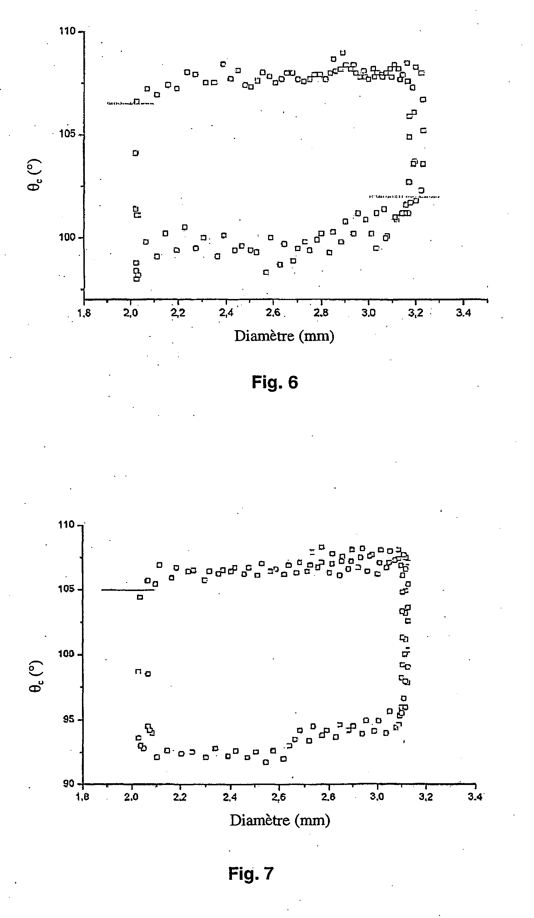

A titre de comparaison, l'angle de mouillage et l'hystérésis de mouillage ont été mesurés (figures 4 à 7) pour des revêtements de surface respectivement constitués :
- d'une couche mince en Téflon® déposée à la tournette (figure 4),
- d'une couche mince en Téflon® déposée par PECVD (figure 5),
- d'une couche mince en SiC_{1,6}O_{1,2}:H déposée par PECVD au moyen d'un précurseur cyclique organo-silane permettant d'obtenir une conformation ou une structure chimique A du composé SiC_{1,6}O_{1,2}:H, notée SiC_{1,6}O_{1,2}:H (A) (figure 6)
- et d'une couche mince en SiC_{1,6}O_{1,2}:H déposée par PECVD au moyen d'un précurseur cyclique organo-silane permettant d'obtenir une conformation ou une structure chimique B du composé SiC_{1,6}O_{1,2}:H, notée SiC_{1,6}O_{1,2}:H (B) et différente de la conformation A (figure 7).

Pour le composé SiC_{1,6}O_{1,2}:H (A), le dépôt est, par exemple, réalisé en introduisant, dans l'enceinte de dépôt, un mélange d'OMCTS et d'hélium. Le mélange est, préalablement réalisé dans une bouteille chauffée à 60°C, au moyen d'un système de bullage d'hélium dont le débit est de l'ordre de 0,2 litre par minute. Le mélange OMCTS/He est ensuite dilué dans de l'hélium, à un débit de 0,632 cm³/min pour être introduit dans l'enceinte. La puissance appliquée sur l'électrode générant le plasma est de 0,02W/cm², la distance interélectrode est de 30mm, la pression à l'intérieur de l'enceinte est maintenue à 0,2mbar au cours du dépôt de la couche mince.

Pour le composé SiC_{1,6}O_{1,2}:H (B), le dépôt est, par exemple, réalisé en introduisant, dans l'enceinte de dépôt, un mélange d'OMCTS et d'hélium. Le mélange est, préalablement réalisé dans une bouteille chauffée à 60°C, au moyen d'un système de bullage d'hélium dont le débit est de l'ordre de 0,4 litre par minute. Le mélange OMCTS/He est ensuite dilué dans de l'hélium, à un début de 0,273 cm³/min pour être introduit dans l'enceinte. La puissance appliquée sur l'électrode générant le plasma est de 0,81W/cm², la distance interélectrode est de 15mm et la pression à l'intérieur de l'enceinte est maintenue à 0,2mbar au cours du dépôt de la couche mince.

Les couches minces en SiC_{1,6}O_{1,2}:H (A) et SiC_{1,6}O_{1,2}:H (B) ont une épaisseur de 1 micromètre.

Les conformations A et B de SiC_{1,6}O_{1,2}:H sont déterminées par spectroscopie infrarouge (FTIR), respectivement illustrées aux figures 2 et 3. L'analyse des spectres infrarouges permet d'obtenir des informations qualitatives et semi-quantitatives sur la nature des liaisons chimiques présentes dans les composés SiC_{1,6}O_{1,2}:H (A) et SiC_{1,6}O_{1,2}:H (B). En effet, chaque pic d'absorption du spectre IR se produit à un nombre d'onde correspondant à un mode de vibration propre à une liaison chimique particulière. Ainsi, le tableau 1 ci-dessous indique, pour chaque pic d'absorption des figures 2 et 3, le mode de vibration correspondant.

**Tableau 1**

| **Référence d'un pic** | **Nombre d'onde** | **Mode de vibration - Liaison** |
|---|---|---|
| **d'absorption** | **(cm⁻¹)** | **correspondante** |
| 1 | 800 | ∂ᵣCH₃ dans Si(CH₃)₂ |
| 2 | 840 | ∂ᵣCH₃ dans Si(CH₃)₃ |
| 3 | 890 | υ SiH |
| 4 | 1020 | υₐ SiOSi linéaire |
| 5 | 1080 | υ SiOSi cyclique |
| 6 | 1130 | υ SiOC dans SiOCH₃ |
| 7 | 1250-1270 | ∂ₛ CH₃ dans Si(CH₃)_{3/2/1} |
| 8 | 2165 | υₛ SiH |
| 9 | 2875 | υₛ CH dans CH₂ |
| 10 | 2905 | υₛ CH dans CH₃ |
| 11 | 2960 | υₛ CH dans CH₃ |

A la figure 2, on constate que le spectre infrarouge du composé SiC_{1,6}O_{1,2}:H (A) comporte deux pics 1 et 7 correspondant à la liaison de type -Si-CH₃ comprise dans les groupements -Si(CH₃)₂ et -Si(CH₃)₃, Par ailleurs, il comporte des pics 4, 5 et 6 correspondant à la liaison chimique de type -Si-O-. Selon le tableau 1, 58,6% des liaisons chimiques -Si-O- sont présentes sous la forme -Si-O-Si-cyclique (pic 5) correspondant à la structure cyclique du précurseur utilisé pour réaliser le dépôt chimique en phase vapeur, 21,2% des liaisons chimiques -Si-O- sont présentes sous la forme -Si-O-Si linéaires correspondant à l'ouverture des cycles du précurseur (pic 4) et 20,2% des liaisons -Si-O-Si sont présentes sous la forme de -Si-O-C- du groupement Si-O-CH₃ (pic 6).

A la figure 3, on constate que le composé SiC_{1,6}O_{1,2}:H (B) est beaucoup plus réticulé que le composé SiC_{1,6}O_{1,2}:H (A), le nombre de groupements -CH₃ (pics 1 et 7) étant plus faible que dans le composé SiC_{1,6}O_{1,2}:H (A). A la place des groupements CH₃, le composé SiC_{1,6}O_{1,2}:H (B) comporte des groupements de type CₓH_{y} (pics 2 et 3). Par ailleurs, la répartition structurelle des liaisons de type -Si-O- est très différente de celle du composé SiC_{1,6}O_{1,2}:H (A). Ainsi, 39,7% des liaisons -Si-O- sont présentent sous la forme -Si-O-Si- linéaires (pic 4), 36,15% sont sous la forme -Si-O-Si- cyclique (pic 5) et 24,15% sont sous la forme -Si-OC- comprise dans le groupement Si-O-CH₃. Le composé SiC_{1,6}O_{1,2}:H (B). comporte également des groupements -Si-H (pics 3 et 8) qui ne sont pas présents dans le composé SiC_{1,6}O_{1,2}:H (A).

Les figures 4 à 7 correspondent à des graphiques mesurant l'angle de contact (θ_{c} en °) en fonction du diamètre (en mm) d'une goutte d'eau déposée sur la surface des quatre revêtements correspondants. Les angles de contact sont mesurés au moyen d'une caméra, en utilisant un système de dépose de goutte d'eau sur la surface du revêtement. A titre d'exemple, le système utilisé est un système automatisé commercialisé par la société Kruss, sous le nom de Drop Shape analysis système DSA 10mk2, permettant non seulement de mesurer l'angle de contact mais aussi l'hystérésis de mouillage en augmentant et en diminuant le volume de la goutte. Une série de mesure permet alors de visualiser le phénomène d'hystérésis de mouillage pour chaque type de revêtement (Téflon, SiC_{1,6}O_{1,2}:H (A) et SiC_{1,6}O_{1,2}:H (B)) et de déterminer, pour chacun, l'angle de contact caractérisant l'hydrophobicité et l'hystérésis de mouillage. Les résultats sont donnés dans le tableau 2 ci-dessous.

**Tableau 2**

| | **Angle de contact (θ_{c} en°)** | **Hystérésis (θᵣ - θₐ)** |
|---|---|---|
| **Téflon - Tournette** | 120° | 12,2° |
| **Téflon - PECVD** | 115° | 25° |
| **SiC_{1,6}O_{1,2}:H (A) de 1µm d'épaisseur - PECVD** | 107° | 4,5 |
| **SiC_{1,6}O_{1,2}:H (B) de 1µm d'épaisseur - PECVD** | 100° | 9,5 |

On constate ainsi que bien que l'hydrophobicité des composés SiC_{1,6}O_{1,2}:H (A) et SiC_{1,6}O_{1,2}:H (B) soit légèrement inférieure à celle des revêtements en Téflon, elle reste, cependant, élevée, l'angle de contact étant supérieur à 100°. Par contre, l'hystérésis de mouillage des composés SiC_{1,6}O_{1,2}:H (A) et SiC_{1,6}O_{1,2}:H (B) est beaucoup plus faible que celui des revêtements en Téflon. Par ailleurs, le composé SiC_{1,6}O_{1,2}:H (A) présente des caractéristiques d'hydrophobicité et d'hystérésis de mouillage plus adaptées au domaine de l'électromouillage sur diélectrique que le composé SiC_{1,6}O_{1,2}:H (B).

De la même manière, l'angle de mouillage et l'hystérésis de mouillage d'un composé de type SiC_{1,3}N_{0,7} : H a été mesuré. L'angle de mouillage est de l'ordre de 92° et l'hystérésis est de 9°. Pour le composé SiC_{1,3}N_{0,7}:H, le dépôt est, par exemple, réalisé en introduisant, dans l'enceinte de dépôt, du TMS dont le débit est de 2cm³/min dilué avec de l'azote dont le débit est de 11/min. La puissance appliquée sur l'électrode générant le plasma est de 300W et la pression à l'intérieur de l'enceinte est maintenue à 0,5mbar au cours de l'étape de dépôt.

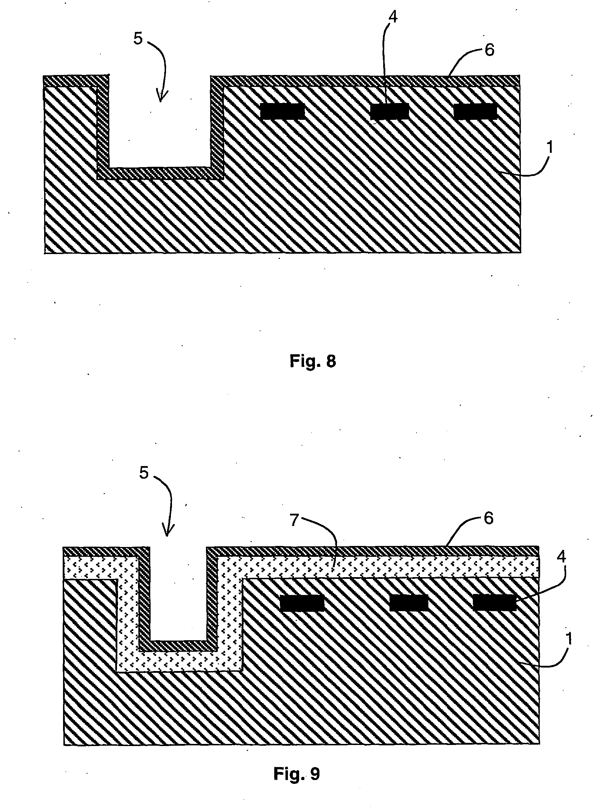

De tels revêtements de surface peuvent être disposés sur la face d'un micro-composant. Ainsi, dans un mode particulier de réalisation représenté à la figure 8, un micro-composant comporte un substrat 1 dont une face est destinée à recevoir au moins une goutte de liquide. Le substrat 1 est muni d'un réseau d'électrodes 4 enterrées à l'intérieur du substrat et d'une cavité 5. Le substrat est recouvert d'un revêtement de surface formé par une couche mince supérieure 6 constituée par un composé choisi parmi SiCₓO_{y}:H et SiC_{x'}N_{y'}:H. La surface libre de la couche mince supérieure 6 est destinée à recevoir la goutte de liquide, de manière à déplacer celle-ci, par exemple depuis la zone du revêtement recouvrant la cavité 5 vers une autre zone du revêtement.

Pour augmenter la constante diélectrique relative du revêtement de surface, une couche mince inférieure diélectrique 7 peut être disposée sous la couche mince supérieure 6, comme représenté à la figure 9. La couche mince inférieure 7 est, de préférence, déposée sur la face du micro-composant, avant la couche mince supérieure 6, soit par dépôt chimique en phase vapeur assisté par plasma (PECVD), soit par pulvérisation magnétron. L'épaisseur de la couche mince inférieure 7 est, de préférence inférieure ou égale à 5 micromètres et la constante diélectrique relative est, de préférence, supérieure ou égale à 2. Plus particulièrement, la couche mince inférieure 7 est, par exemple, constituée d'un composé choisi parmi le groupe comprenant SiO₂, SiN, SiCN, Ta₂O₅, TiO₂, HfO₂, ZrO₂, Al₂O₃, SrTiO₃, BaTiO₃ et Pb(ZrTi)O₃. A titre d'exemple, le dépôt PECVD de la couche mince inférieure est, par exemple, réalisé sous excitation radiofréquence (RF) avec un précurseur de type SiH₄/N₂O tandis que la pulvérisation magnétron peut être réalisée avec une cible en Si₃N₄. Une telle couche mince inférieure 7 permet de renforcer la constante diélectrique relative du revêtement de surface et augmente encore la tenue au claquage.

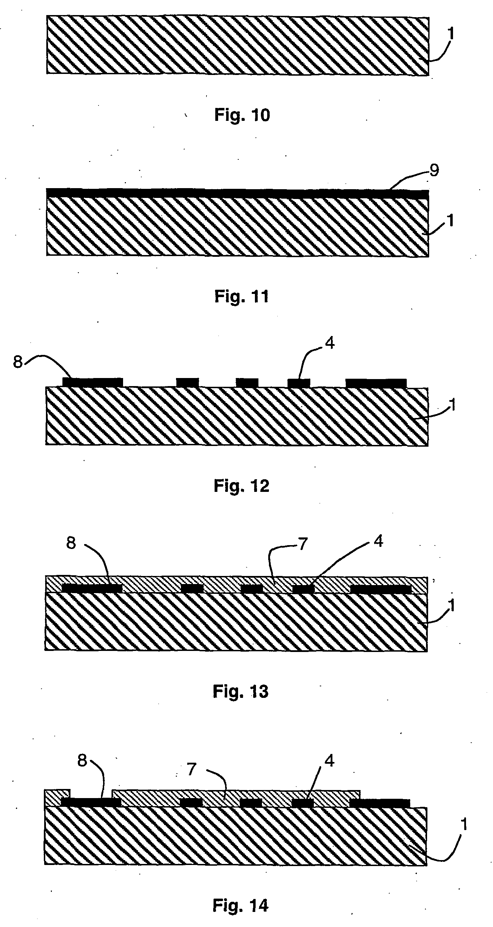

Dans un mode particulier de réalisation illustré aux figures 10 à 16, un micro-composant est réalisé à partir d'un substrat 1 tel que représenté à la figure 10 et composé, par exemple de verre, de silice, de polycarbonate ou de silicium recouvert d'une couche isolante telle que l'oxyde de silicium. Un réseau d'électrodes 4 connectées à des plots 8 est ensuite formé à la surface du substrat 1 (figure 12), en déposant préalablement une couche mince métallique 9 (figure 11) à la surface du substrat 1 puis en la structurant par photolithographie. La couche mince métallique 9 peut être, par exemple, en or, en aluminium, en titane, en platine ou en oxyde d'indium et d'étain et son épaisseur est, de préférence, de l'ordre de 0,1 µm à 2µm.

De tels micro-composants peuvent être utilisés pour déplacer une goutte disposée sur la surface libre du revêtement, en appliquant successivement une tension aux différentes électrodes.

Le substrat 1 muni du réseau d'électrodes 4 est ensuite recouvert d'une couche mince inférieure 7 (figure 13) telle qu'une couche d'oxyde de silicium ou une couche de nitrure de silicium, d'une épaisseur de 0,1 µm à 5µm. La couche mince inférieure 7 est ensuite gravée localement, au niveau des plots 8 pour réaliser la reprise de contact électrique (figure 14). Ainsi, la gravure localisée permet de libérer sélectivement une partie seulement de la surface des plots 8 et le reste de la face libre du micro-composant, constitué par les zones non libérées des plots 8, la surface libre du substrat 1 et les électrodes 4, reste recouvert par la couche mince inférieure 7.

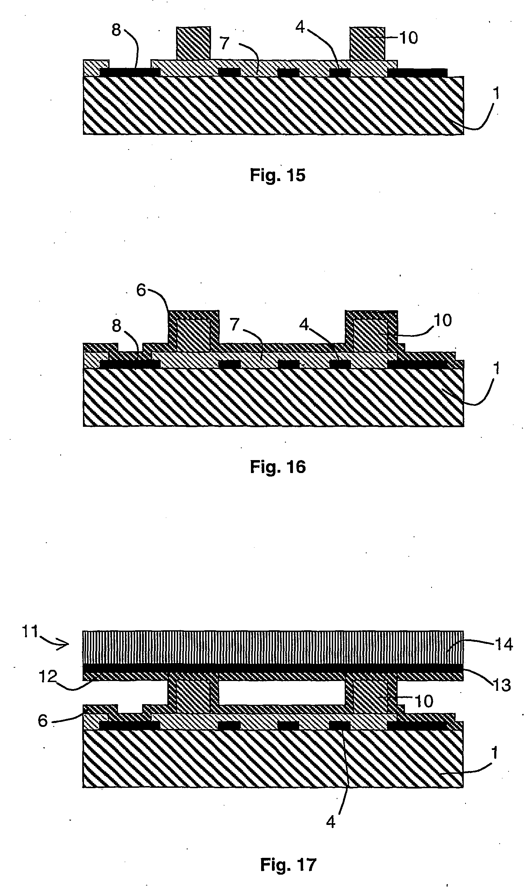

Une couche en résine photosensible telle que la résine SU8 est, ensuite, déposée sur la face libre du micro-composant et elle est structurée par photolithographie, de manière à former des parois (ou motifs) 10 (figure 15) qui peuvent, par exemple, être utilisées comme parois d'un canal ou d'une cavité, dans un composant micro-fluidique. Les parois (ou motifs) 10 ont, par exemple, une épaisseur comprise entre quelques dizaines de micromètres et quelques centaines de micromètres.

Une couche mince supérieure 6, d'une épaisseur comprise entre 0,1 µm et 5µm, et avantageusement d'une épaisseur de 1µm, est ensuite déposée, par dépôt PECVD, sur la totalité de la surface libre du micro-composant (figure 16). Elle est constituée par un matériau choisi parmi SiCₓO_{y}:H et SiC_{x'}N_{y'}:H. Ainsi, la couche mince supérieure 6, formant le revêtement de surface hydrophobe et à faible hystérésis, recouvre totalement la couche mince inférieure 7 ainsi que la partie libre des plots 8 et l'ensemble des parois 10. Le substrat peut ensuite être découpé de manière à former des puces individuelles, connectées électriquement.

Selon une variante de réalisation représentée à la figure 17, le micro-composant représenté à la figure 16 peut comporter un capot 11 reposant sur les parois 10, de manière à former un canal ou une cavité. Le capot 11 comporte alors une couche mince 12 hydrophobe, une couche conductrice 13 et une couche de protection 14, par exemple en polycarbonate, en verre ou en silicium. La couche mince hydrophobe 12 est, de préférence, constituée par un composé choisi parmi SiCₓO_{y}:H et SiC_{x'}N_{y'}:H et la couche conductrice 13 peut être en or, en aluminium, en titane, en platine ou en oxyde d'indium et d'étain. La couche hydrophobe 12 forme, alors un second revêtement de surface délimitant la paroi supérieure du canal ou de la cavité, de sorte que la totalité des parois du canal ou de la cavité est hydrophobe et à faible hystérésis de mouillage.

Selon l'invention, la couche supérieure d'un revêtement de surface est constituée par une sélection particulière de composés choisis parmi SiCₓO_{y}:H avec x compris entre 1,4 et 2 et y compris entre 0,8 et 1,4 et SiC_{x'}N_{y'}:H avec x' compris entre 1,2 et 1,4 et y' compris entre 0,6 et 0,8. Cette sélection permet d'obtenir un revêtement qui est, à la fois hydrophobe et à faible hystérésis de mouillage, contrairement à d'autres composés déjà connus. A titre d'exemple, la demande de brevet EP-A-0289402 décrit, de manière générale, un revêtement protecteur en composé inorganique du type SiCₓN_{y}O_{z}Hₜ, avec x compris entre 0 et 5 et plus particulièrement entre 0,3 et 1, y compris entre 0 et 0,8 et plus particulièrement entre 0,5 et 0,8, z compris entre 0 et 2,5 et plus particulièrement entre 1,3 et 2 et t compris entre 0 et 1,2 et plus particulièrement entre 0,6 et 1. Ce composé inorganique est obtenu par traitement avec un plasma et il est destiné à recouvrir un substrat polymère transparent, en particulier pour la protection mécanique et anti-statique de vitrages, écrans ou supports optiques. Le composé choisi pour constituer la couche supérieure d'un revêtement de surface selon l'invention se différencie du revêtement décrit dans la demande de brevet EP-A-0289402 dans la mesure où le domaine des proportions en carbone, oxygène ou azote et hydrogène selon l'invention est étroit par rapport à celui décrit dans la demande de brevet EP-A-0289402, il est également éloigné du mode de réalisation préféré décrit dans la demande de brevet EP-A-0289402. La sélection des proportions en oxygène ou en azote et en carbone est réalisée non pas de manière arbitraire mais dans le but d'obtenir un revêtement hydrophobe et à faible hystérésis de mouillage.

Par ailleurs, dans l'article "Improvement of hardness in plasma polymerized hexamethyldisiloxane coatings by silica-like surface modification" (Thin, Solid Films 377-378 (2000) 109-114), F. Benitez et al. étudient les revêtements à base d'hexaméthyldisiloxane (HMDSO) polymérisé par plasma (PPHMDSO) dans le but d'améliorer la protection contre la corrosion des substrats en verre recouverts d'une couche en aluminium. Les revêtements étudiés sont obtenus à partir de différents mélanges de gaz constitués de vapeur de monomère HMDSO et d'oxygène ou bien en déposant un monomère pur et en réalisant une oxydation après dépôt, par traitement plasma et des mesures de l'angle de contact d'un film réalisé dans ces différentes conditions sont rapportées. Le seul cas où l'angle de contact est supérieur à 100° est celui où la pression relative en oxygène est nulle. Par ailleurs, l'analyse des pics IR relatifs aux groupements -CH3 indique qu'il ne reste aucune liaison -CH3 au-delà de 35% en oxygène dans le plasma. Cependant, F. Benitez et al. ne mentionnent pas la teneur en carbone ni les proportions spécifiques en oxygène ou en azote, et en hydrogène, qu'il faudrait utiliser pour obtenir un revêtement qui serait non seulement hydrophobe mais également à faible hystérésis de mouillage.

## Revendications

1. Revêtement de surface hydrophobe **caractérisé en ce qu'**il comporte au moins une couche mince supérieure (6, 12) constituée par un composé choisi parmi SiCₓO_{y}:H avec x compris entre 1,4 et 2 et y compris entre 0,8 et 1,4 et SiC_{x'}N_{y'}:H avec x' compris entre 1,2 et 1,4 et y' compris entre 0,6 et 0,8, de manière à obtenir une surface libre à faible hystérésis de mouillage.

2. Revêtement de surface selon la revendication 1, **caractérisé en ce que** la couche mince supérieure (6, 12) a une épaisseur inférieure ou égale à 1 micromètre.

3. Revêtement de surface selon l'une des revendications 1 et 2, **caractérisé en ce que** le revêtement de surface comporte une couche mince inférieure diélectrique (7).

4. Revêtement de surface selon la revendication 3, **caractérisé en ce que** la couche mince inférieure (7) a une épaisseur inférieure ou égale à 5 micromètres.

5. Revêtement de surface selon l'une des revendications 3 et 4, **caractérisé en ce que** la couche mince inférieure (7) a une constante diélectrique relative supérieure ou égale à 2.

6. Revêtement de surface selon l'une quelconque des revendications 3 à 5, **caractérisé en ce que** la couche mince inférieure (7) est constituée d'un composé choisi parmi le groupe comprenant SiO₂, SiN, SiCN, Ta₂O₅, TiO₂, HfO₂, ZrO₂, Al₂O₃, SrTiO₃, BaTiO₃ et Pb(ZrTi)O₃.

7. Procédé de dépôt d'un revêtement de surface selon l'une quelconque des revendications 1 à 6, sur une face d'un micro-composant, **caractérisé en ce qu'**il consiste au moins à réaliser un dépôt chimique en phase vapeur sur la face dudit micro-composant, pour former une couche mince supérieure (6, 12) constituée par un composé choisi parmi SiCₓO_{y}:H avec x compris entre 1,4 et 2 et y compris entre 0,8 et 1,4 et SiC_{x'}N_{y'}:H, avec x' compris entre 1,2 et 1,4 et y' compris entre 0,6 et 0,8, le dépôt chimique en phase vapeur étant réalisé au moyen d'un précurseur choisi parmi les organo-silanes.

8. Procédé de dépôt selon la revendication 7, **caractérisé en ce que** le dépôt chimique en phase vapeur est un dépôt chimique en phase vapeur assisté par plasma.

9. Procédé de dépôt selon la revendication 8, **caractérisé en ce que** le dépôt chimique en phase vapeur assisté par plasma est réalisé en mode pulsé.

10. Procédé de dépôt selon l'une des revendications 7 à 9, **caractérisé en ce que** la couche mince supérieure (6,12) étant en SiCₓO_{y}:H, le précurseur organo-silane est constitué par l'octaméthylcyclotétrasiloxane et il est mélangé avec de l'hélium.

11. Procédé de dépôt selon l'une des revendications 7 à 9, **caractérisé en ce que** la couche mince supérieure (6, 12) étant en SiC_{x'}N_{y'}:H, le précurseur organo-silane est constitué par le trétraméthylsilane et il est mélangé avec de l'azote.

12. Procédé de dépôt selon l'une des revendications 7 à 11, **caractérisé en ce qu'**il consiste à déposer sur la face dudit micro-composant, avant la couche mince supérieure (6, 12), une couche mince inférieure diélectrique (7), par dépôt chimique en phase vapeur assisté par plasma ou par pulvérisation magnétron.

13. Micro-composant comportant au moins un substrat (1) muni d'un réseau d'électrodes (4) et dont une face est destinée à recevoir au moins une goutte de liquide, micro-composant **caractérisé en ce qu'**il comporte un revêtement de surface selon l'une des revendications 1 à 6, disposé sur la face du micro-composant de manière à ce que la surface libre du revêtement reçoive la goutte.

14. Utilisation du micro-composant selon la revendication 13, **caractérisé en ce qu'**une tension est appliquée successivement aux différentes électrodes afin de déplacer la goutte disposée sur la surface libre du revêtement.

## Claims

1. Hydrophobic surface coating **characterized in that** it comprises at least an upper thin layer (6, 12) formed by a compound selected among SiCₓO_{y}:H with x comprised between 1.4 and 2 and y comprised between 0.8 and 1.4 and SiC_{x'}N_{y'}:H with x' comprised between 1.2 and 1.4 and y' comprised between 0.6 and 0.8, so as to obtain a free surface with a low wetting hysteresis.

2. Surface coating according to claim 1, **characterized in that** the upper thin layer (6, 12) has a thickness less than or equal to 1 micrometer.

3. Surface coating according to one of claims 1 and 2, **characterized in that** the surface coating comprises a dielectric bottom thin layer (7).

4. Surface coating according to claim 3, **characterized in that** the bottom thin layer (7) has a thickness less than or equal to 5 micrometers.

5. Surface coating according to one of claims 3 and 4, **characterized in that** the bottom thin layer (7) has a relative dielectric constant greater than or equal to 2.

6. Surface coating according to any one of claims 3 to 5, **characterized in that** the bottom thin layer (7) is formed by a compound selected from the group comprising SiO₂, SiN, SiCN, Ta₂O₅, TiO₂, HfO₂, ZrO₂, Al₂O₃, SrTiO₃, BaTiO₃ and Pb(ZrTi)O₃.

7. Method for depositing a surface coating according to any one of claims 1 to 6 on a surface of a microcomponent, **characterized in that** it consists at least in performing chemical vapor deposition on the surface of said microcomponent to form an upper thin layer (6, 12) formed by a compound selected among SiCₓO_{y}:H with x comprised between 1.4 and 2 and y comprised between 0.8 and 1.4 and SiC_{x'}N_{y'}:H, with x' comprised between 1.2 and 1.4 and y' comprised between 0.6 and 0.8, the chemical vapor deposition being performed by means of a precursor selected among organosilanes.

8. Method for depositing according to claim 7, **characterized in that** the chemical vapor deposition is plasma enhanced chemical vapor deposition.

9. Method for depositing according to claim 8, **characterized in that** the plasma enhanced chemical vapor deposition is performed in pulsed mode.

10. Method for depositing according to one of claims 7 to 9, **characterized in that** the upper thin layer (6, 12) being made of SiCₓO_{y}:H, the organosilane precursor is formed by octamethylcyclotetrasiloxane and mixed with helium.

11. Method for depositing according to one of claims 7 to 9, **characterized in that** the upper thin layer (6, 12) being made of SiC_{x'}N_{y'}:H, the organosilane precursor is formed by tetramethylsilane and mixed with nitrogen.

12. Method for depositing according to one of claims 7 to 11, **characterized in that** it consists in depositing a bottom dielectric thin layer (7) on the surface of said microcomponent, before forming the upper thin layer (6, 12), by plasma enhanced chemical vapor deposition or by magnetron sputtering.

13. Microcomponent comprising at least a substrate (1) provided with an electrodes array (4) and having a surface designed to receive at least a drop of liquid, microcomponent **characterized in that** it comprises a surface coating according to one of claims 1 to 6 arranged on the surface of the microcomponent in such a way that the free surface of the coating receives the drop.

14. Use of the microcomponent according to claim 13, **characterized in that** a voltage is successively applied to the different electrodes so as to move the drop arranged on the free surface of the coating.

## Patentansprüche

1. Hydrophobe Oberflächenbeschichtung, **dadurch gekennzeichnet, dass** sie mindestens eine obere Dünnschicht (6, 12) umfasst, die von einer Verbindung gebildet wird, die ausgewählt ist aus SiCₓO_{y}:H, mit x zwischen 1,4 und 2 und y zwischen 0,8 und 1,4, und SiC_{x'}N_{y'}:H, mit x' zwischen 1,2 und 1,4 und y' zwischen 0,6 und 0,8, sodass man eine freie Oberfläche mit geringer Benetzungshysterese erhält.

2. Oberflächenbeschichtung nach Anspruch 1, **dadurch gekennzeichnet, dass** die obere Dünnschicht (6, 12) eine Dicke von unter oder gleich 1 Mikrometer hat.

3. Oberflächenbeschichtung nach einem der Ansprüche 1 und 2, **dadurch gekennzeichnet, dass** die Oberflächenbeschichtung eine untere dielektrische Dünnschicht (7) aufweist.

4. Oberflächenbeschichtung nach Anspruch 3, **dadurch gekennzeichnet, dass** die untere Dünnschicht (7) eine Dicke von unter oder gleich 5 Mikrometer hat.

5. Oberflächenbeschichtung nach einem der Ansprüche 3 und 4, **dadurch gekennzeichnet, dass** die untere Dünnschicht (7) eine relative Dielektrizitätskonstante von über oder gleich 2 hat.

6. Oberflächenbeschichtung nach einem der Ansprüche 3 bis 5, **dadurch gekennzeichnet, dass** die untere Dünnschicht (7) von einer Verbindung gebildet wird, die ausgewählt ist aus der Gruppe, die SiO₂, SiN, SiCN, Ta₂O₅, TiO₂, HfO₂, ZrO₂, Al₂O₃, SrTiO₃, BaTiO₃ und Pb(ZrTi)O₃ umfasst.

7. Verfahren zum Abscheiden einer Oberflächenbeschichtung nach einem der Ansprüche 1 bis 6 auf einer Seite eines Mikrobauteils, **dadurch gekennzeichnet, dass** es mindestens darin besteht, dass eine Gasphasenabscheidung auf der Seite des Mikrobauteils erfolgt, zur Bildung einer oberen Dünnschicht (6, 12), die von einer Verbindung gebildet wird, die ausgewählt ist aus SiCₓO_{y}:H, mit x zwischen 1,4 und 2 und y zwischen 0,8 und 1,4, und SiC_{x'}N_{y'}:H, mit x' zwischen 1,2 und 1,4 und y' zwischen 0,6 und 0,8, wobei die Gasphasenabscheidung mittels eines Precursors erfolgt, der aus den Organosilanen ausgewählt ist.

8. Abscheidungsverfahren nach Anspruch 7, **dadurch gekennzeichnet, dass** die Gasphasenabscheidung eine plasmaunterstützte Gasphasenabscheidung ist.

9. Abscheidungsverfahren nach Anspruch 8, **dadurch gekennzeichnet, dass** das plasmaunterstützte CVD-Verfahren im Impulsbetrieb durchgeführt wird.

10. Abscheidungsverfahren nach einem der Ansprüche 7 bis 9, **dadurch gekennzeichnet, dass**, wenn die obere Dünnschicht (6, 12) von SiCₓO_{y}:H gebildet wird, der Organosilan-Precursor Octamethylcyclotetrasiloxan ist und mit Helium gemischt wird.

11. Abscheidungsverfahren nach einem der Ansprüche 7 bis 9, **dadurch gekennzeichnet, dass**, wenn die obere Dünnschicht (6, 12) von SiC_{x'}N_{y'}:H gebildet wird, der Organosilan-Precusor Tetramethylsilan ist und mit Stickstoff gemischt wird.

12. Abscheidungsverfahren nach einem der Ansprüche 7 bis 11, **dadurch gekennzeichnet, dass** es darin besteht, auf die Seite des Mikrobauteils vor der oberen Dünnschicht (6, 12) eine untere dielektrische Dünnschicht (7) mittels plasmaunterstütztem CVD-Verfahren oder Magnetronzerstäubung aufzubringen.

13. Mikrobauteil, das mindestens ein Substrat (1) umfasst, das mit einem Netz aus Elektroden (4) versehen ist und von dem eine Seite mindestens einen Flüssigkeitstropfen aufnehmen soll, Mikrobauteil, das **dadurch gekennzeichnet ist, dass** es eine Oberflächenbeschichtung nach einem der Ansprüche 1 bis 6 umfasst, die auf die Seite des Mikrobauteils in der Weise aufgebracht ist, dass die freie Fläche der Beschichtung den Tropfen aufnimmt.

14. Verwendung des Mikrobauteils nach Anspruch 13, **dadurch gekennzeichnet, dass** die einzelnen Elektroden nacheinander mit einer Spannung beaufschlagt werden, um den auf der freien Fläche der Beschichtung angeordneten Tropfen fortzubewegen.
